(19) Europäisches Patentamt
European Patent Office
Office européen des brevets

(11) Veröffentlichungsnummer : **0 103 796**
**B1**

(12) # EUROPÄISCHE PATENTSCHRIFT

(45) Veröffentlichungstag der Patentschrift : **11.11.87**

(51) Int. Cl.⁴ : **C 07 D239/20**, A 61 K 31/505

(21) Anmeldenummer : **83108692.1**

(22) Anmeldetag : **03.09.83**

(54) **Neue Dihydropyrimidine, Verfahren zu ihrer Herstellung sowie ihre Verwendung in Arzneimitteln.**

(30) Priorität : **18.09.82 DE 3234684**

(43) Veröffentlichungstag der Anmeldung : **28.03.84 Patentblatt 84/13**

(45) Bekanntmachung des Hinweises auf die Patenterteilung : **11.11.87 Patentblatt 87/46**

(84) Benannte Vertragsstaaten : **AT BE CH DE FR GB IT LI NL SE**

(56) Entgegenhaltungen :
**CHEMICAL ABSTRACTS, Band 75, 1971, Seite 217, Nr. 47266e, COLUMBUS OHIO (US), A. ZIDERMANE et al.: "Antitumor action of dihydropyridine and dihydropyrimidine derivatives"**

(73) Patentinhaber : **BAYER AG**
**Konzernverwaltung RP Patentabteilung**
**D-5090 Leverkusen 1 Bayerwerk (DE)**

(72) Erfinder : **Stoltefuss, Jürgen, Dipl.-Ing.**
**Parkstrasse 20**
**D-5657 Haan (DE)**
Erfinder : **Böshagen, Horst, Dr.**
**Wiesenstrasse 4**
**D-5657 Haan (DE)**
Erfinder : **Schramm, Matthias, Dr.**
**Paffrather Strasse 38**
**D-5000 Köln 80 (DE)**
Erfinder : **Thomas, Günter, Dr.**
**Claudiusweg 9**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft neue Dihydropyrimidine, Verfahren zu ihrer Herstellung sowie ihre Verwendung in Arzneimitteln, insbesondere in kreislaufbeeinflussenden Arzneimitteln.

Es ist bereits bekanntgeworden, daß man aus Benzamidin und α,β-ungesättigten Ketonen Dihydropyrimidine erhalten kann (vgl. Silbersmith, J. Org. Chem. 27, 4090 (1982)). Es ist aber auch bekannt, daß Benzylidenmalonsäureester mit Amidinen 3,4-Dihydropyridone-2 ergeben (DE 2 242 787).

Es ist weiterhin bekannt, daß bestimmte Dihydropyrimidine Antitumor-Wirkungen bei Ratten und Mäusen zeigen. Diese Dihydropyrimidine unterscheiden sich von den erfindungsgemäßen Verbindungen sowohl in ihrer Wirkung als auch in ihrer chemischen Struktur, da sie in 2-Position durch Hydroxy- oder Mercapto-Gruppen substitutiert sind (vergl. A. Zidermane et al. : C. A. 75, (1971), Nr. 47266e, Seite 217).

Die Erfindung betrifft neue Dihydropyrimidine der allgemeinen Formel (I)

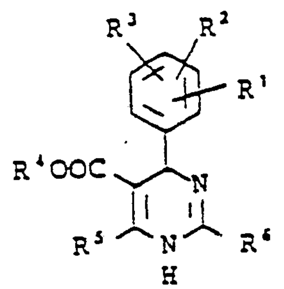

(I)

bzw. deren mesomere Form Ia

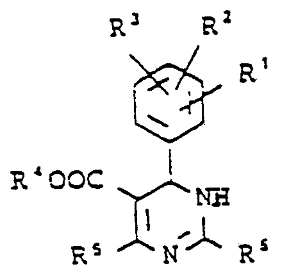

(Ia)

oder ein Gleichgewicht zwischen beiden Formen, in welcher $R^1$, $R^2$ und $R^3$ gleich oder verschieden sind und jeweils für Wasserstoff, Alkyl mit 1-4 Kohlenstoffatomen, Halogen, Nitro, Cyano, Hydroxy, Amino, Alkoxy mit 1-4 Kohlenstoffatomen, Benzyl, Monoalkylamino oder Dialkylamino mit jeweils 1-4 Kohlenstoffatomen in den Alkylresten oder für Halogenalkyl mit 1-4 Kohlenstoffatomen, oder für den Rest

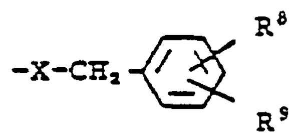

stehen, in welchem

X O, S oder $SO_2$ bedeutet und

$R^8$ und $R^9$ gleich oder verschieden sind und jeweils für Wasserstoff, Nitro, Trifluormethyl, Halogen, Alkyl oder Alkoxy mit jeweils 1-4 Kohlenstoffatomen stehen und

$R^4$ für Wasserstoff oder einen geradkettigen, verzweigten oder cyclischen Alkyl- oder Alkenylrest steht, der gegebenenfalls ein oder zwei gleiche oder verschiedene Heterokettenglieder aus der Gruppe O, CO, NH, N-Alkyl mit 1-4 Kohlenstoffatomen, S oder $SO_2$ enthält und der gegebenenfalls substituiert ist durch Halogen, Nitro, Cyano, Hydroxy, Phenyl, Benzyl, Pyridyl, Amino, Monoalkylamino oder Dialkylamino mit jeweils 1 bis 4 Kohlenstoffatomen in den Alkylresten und

$R^5$ für einen geradkettigen, verzweigten oder cyclischen Alkyl- oder Alkenylrest mit bis zu 10 Kohlenstoffatomen steht, der gegebenenfalls substituiert ist durch Halogen, Hydroxy, Cyano, Amino, Alkoxy, Alkylthio, Alkoxycarbonyl, Monoalkylamino, Dialkylamino mit jeweils 1-4 Kohlenstoffatomen in den Alkyl- und Alkoxyresten, Phenyloxy, Benzylamino oder durch einen 5- bis 7-gliedrigen heterocyclischen Ring der ein oder zwei Heteroatome aus der Gruppe Sauerstoff oder Stickstoff als Ringglieder enthält, und

$R^6$ für Wasserstoff, einen geradkettigen, verzweigten oder cyclischen Alkyl- oder Alkenylrest mit bis zu 10 Kohlenstoffatomen steht der gegebenenfalls durch Halogen, Hydroxy, Cyano, Nitro, Amino, Alkoxy,

Alkylthio, Alkoxycarbonyl, Monoalkylamino oder Dialkylamino mit jeweils 1-4 Kohlenstoffatomen in den Alkyl- und Alkoxyresten oder durch Phenoxy substituiert ist, oder für einen Phenyl-, Naphthyl-, Benzyl- oder Phenethylrest steht oder für einen stickstoffhaltigen Heteroarylrest steht, wobei die Arylreste gegebenenfalls 1- oder 2-fach substituiert sind durch Halogen, Hydroxy, Cyano, Amino, Nitro, Carboxy, Alkoxy, Alkylthio, Carbalkoxy, Acyloxy, Monoamino oder Dialkylamino mit jeweils bis zu 4 Kohlenstoffatomen in den Alkyl-, Alkoxy- und Acylresten, sowie ihre pharmakologisch unbedenklichen Additionssalze.

Die neuen erfindungsgemäßen Dihydropyrimidine der allgemeinen Formel (I) bzw. (Ia) besitzen wertvolle pharmazeutische Eigenschaften. Aufgrund ihrer kreislaufbeeinflussenden Wirkung können sie als antihypertensive Mittel, als Vasodilatatoren, als Cerebraltherapeutika sowie als Coronartherapeutika Verwendung finden.

Die erfindungsgemäßen Verbindungen der allgemeinen Formel (I) bzw. (Ia) können hergestellt werden, indem man

A) Yliden-β-ketoester der allgemeinen Formel (II)

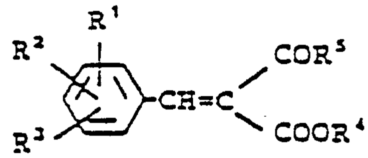

(II)

in welcher $R^1$, $R^2$, $R^3$, $R^4$ und $R^5$ die oben angegebene Bedeutung haben, mit Amidinen der allgemeinen Formel (III)

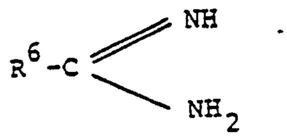

(III)

in welcher $R^6$ die oben angegebene Bedeutung hat, gegebenenfalls in Gegenwart inerter organischer Lösungsmittel bei Temperaturen zwischen 20° und 150° C mit oder ohne Basen- oder Säurezusatz umsetzt, oder

B) Aldehyde der allgemeinen Formel (IV)

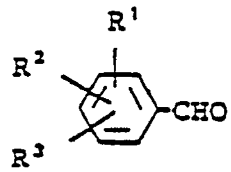

(IV)

in welcher $R^1$, $R^2$ und $R^3$ die oben angegebene Bedeutung haben, mit Enaminocarbonsäureestern der allgemeinen Formel (V)

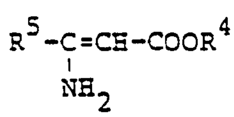

(V)

in welcher $R^4$ und $R^5$ die oben angegebene Bedeutung haben, und Amidinen der allgemeinen Formel (III) wie oben beschrieben umsetzt, oder

C) Aldehyde der allgemeinen Formel (IV)

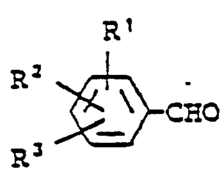

(IV)

in welcher $R^1$, $R^2$ und $R^3$ die oben angegebene Bedeutung haben, mit Amidinen der Formel (III)

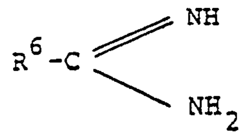

in welcher $R^6$ die oben angegebene Bedeutung hat und β-Ketocarbonsäureestern der allgemeinen Formel (VI)

$$R^5—CO—CH_2—COOR^4 \qquad (VI)$$

in welcher $R^4$ und $R^5$ die oben angegebene Bedeutung haben, gegebenenfalls in Gegenwart inerter organischer Lösungsmittel mit oder ohne Basen- bzw. Säurezusatz umsetzt.

Setzt man nach Verfahrensvariante A) 3-Chlorbenzylidenacetessigsäuremethylester mit Acetamidin um, so läßt sich die Reaktion durch folgendes Formelschema wiedergeben :

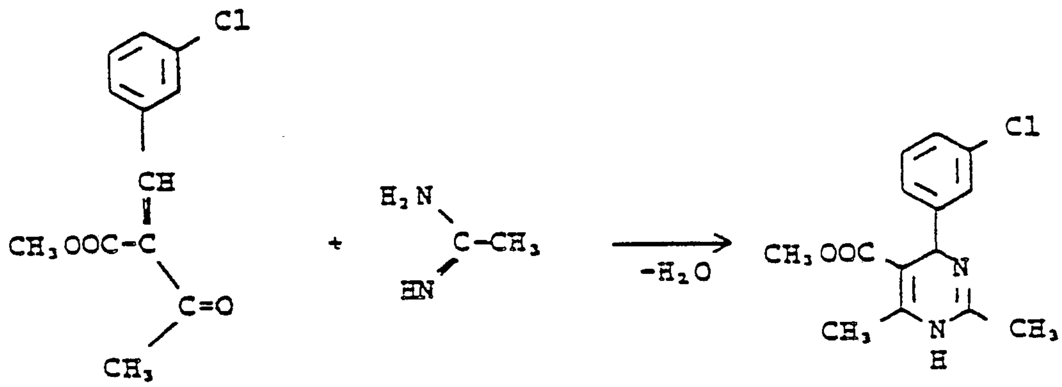


Setzt man nach Verfahrensvariante B) 3-Chlorbenzaldehyd mit β-Aminocrotonsäuremethylester und Benzamidin um, so läßt sich die Reaktion durch folgendes Formelschema wiedergeben :

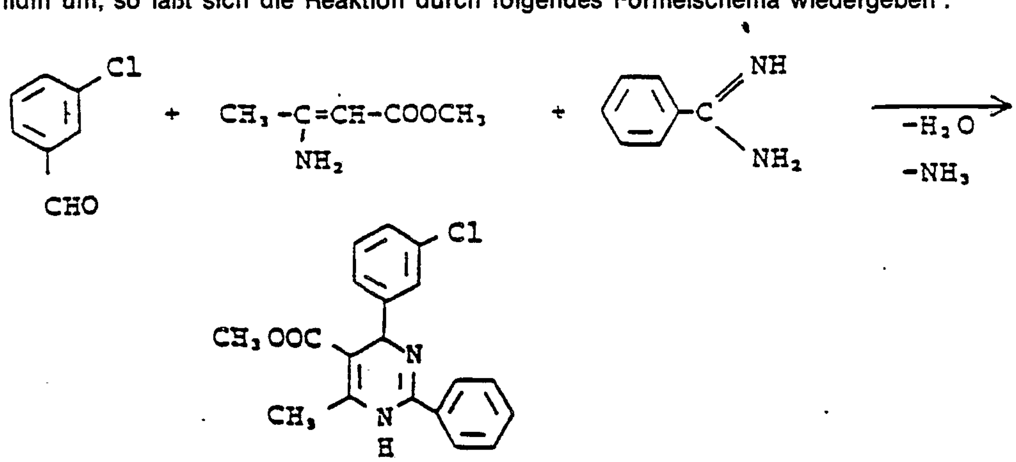


Setzt man nach Verfahrensvariante C) 2-Trifluormethylbenzaldehyd mit Formamidin und Acetessigsäureethylester um, so läßt sich die Reaktion durch folgendes Formelschema wiedergeben :

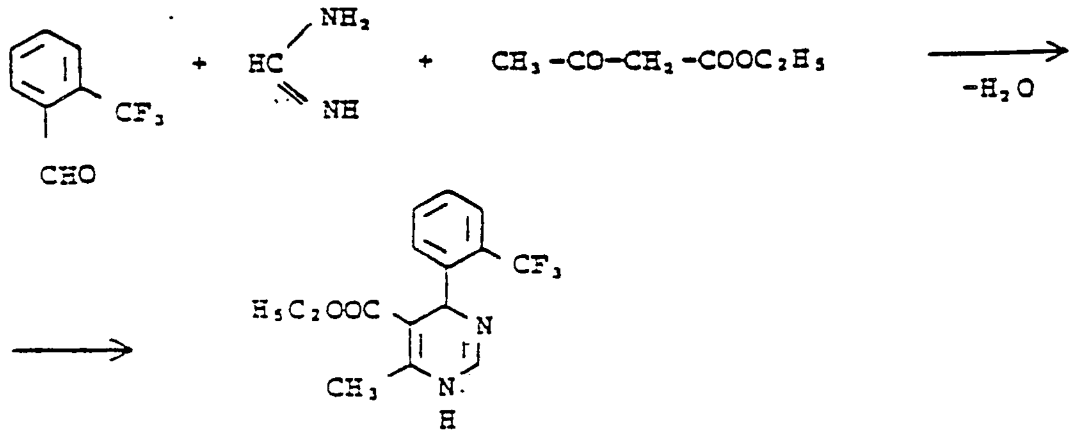

Die als Ausgangsstoffe verwendeten Yliden-β-ketoester der Formel (II) können nach literaturbekannten Methoden dargestellt werden (vgl. G. Jones, « The Knoevenagel Condensation », in Org. Reactions, Vol. XV, 204 ff (1967)).

Die als Ausgangsstoffe verwendeten Enaminocarbonsäureester (V) sind bekannt oder können nach literaturbekannten Methoden dargestellt werden (vgl. A. C. Cope, J. Am. Chem. Soc. 67, 1017 (1945)).

Die als Ausgangsstoffe verwendeten β-Ketocarbonsäureester (VI) sind bekannt oder können nach literaturbekannten Methoden dargestellt werden (z. B. D. Borrmann, « Umsetzung von Diketen mit Alkoholen, Phenolen und Mercaptanen », in Houben-Weyl, Methoden der organischen Chemie, Vol. VII/4, 230 ff (1968) ; Y. Oikawa, K. Sugano und O. Yonemitsu, J. Org. Chem. 43, 2087 (1978)).

Die als Ausgangsstoffe verwendeten Aldehyde (IV) sind bekannt oder können nach literaturbekannten Methoden dargestellt werden (vgl. T. D. Harris und G. P. Roth, J. Org. Chem. 44, 146, (1979), Deutsche Offenlegungsschrift 2 165 260, Juli 1972, Deutsche Offenlegungsschrift 2 401 665, Juli 1974, Mijano et al., Chem. Abst. 59 (1963), 13 929 c, E. Adler und H.-D. Becker, Chem. Scand. 15, 849 (1961), E. P. Papadopoulos, M. Mardin und Ch. Issidoridis, J. Org. Chem. 31, 615 (1966), J. A. Chem. Soc. 78, 2543 (1956)).

Die als Ausgangsstoffe verwendeten Amidine (III) sind bekannt oder können nach literaturbekannten Methoden hergestellt werden (vgl. Houben-Weyl, Methoden der organischen Chemie, Vol. 11/2, Seite 38 ff (1958) ; R. L. Shoiner und F. W. Neumann, Chem. Reviews 35, 351 (1944)).

Für alle Verfahrensvarianten A, B und C kommen als Verdünnungsmittel alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Die Umsetzung kann mit oder ohne Basen- bzw. Säurezusatz durchgeführt werden, es hat sich jedoch gezeigt, daß eine Umsetzung im Sinne der Erfindung vorzugsweise in Gegenwart von schwächeren Säuren wie z. B. Essigsäure oder Ameisensäure stattfindet.

Von besonderem Interesse sind Dihydropyrimidine der allgemeinen Formel (I) in welcher.

$R^1$, $R^2$ und $R^3$ gleich oder verschieden sind und für Wasserstoff, Alkyl mit 1-4 Kohlenstoffatomen, Fluor, Chlor, Nitro, Trifluormethyl, oder für den Rest

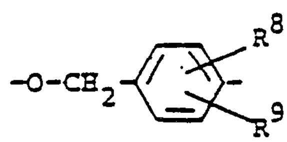

stehen, in welchem

$R^8$ und $R^9$ gleich oder verschieden sind und für Wasserstoff, Alkyl mit 1 bis 4 Kohlenstoffatomen, Fluor, Chlor, Nitro oder Trifluormethyl stehen,

$R^4$ und $R^5$ gleich oder verschieden sind und für Alkyl mit 1-6 Kohlenstoffatomen stehen, das gegebenenfalls in der Kette durch ein Sauerstoff unterbrochen ist und gegebenenfalls substituiert ist durch Fluor, Chlor, Cyano, Nitro und Phenyl, und

$R^6$ für Wasserstoff, Alkyl mit 1-4 Kohlenstoffatomen, Phenyl oder Pyridyl steht, sowie ihre pharmakologisch unbedenklichen Additionssalze.

Die vorstehenden Herstellungsverfahren sind lediglich zur Verdeutlichung angegeben, und die Herstellung der Verbindungen der Formel (I) bzw. (Ia) ist nicht auf diese Verfahren beschränkt, sondern jede Modifikation dieser Verfahren ist in gleicher Weise für die Herstellung der erfindungsgemäßen Verbindungen anwendbar.

Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E. L. Eliel, Stereochemistry of Carbon Compounds, McGraw Hill, 1962).

Die Verbindungen der allgemeinen Formel (I) bzw. (Ia) zeigen interessante biologische Wirkungen. Sie besitzen ein breites und vielseitiges pharmakologisches Wirkungsspektrum und können als Coronarmittel und Blutdruckmittel eingesetzt werden. Im einzelnen seien folgende Hauptwirkungen genannt :

1. Die Verbindungen bewirken bei parenteraler, oraler und perlingualer Zugabe eine deutliche Erweiterung der Coronargefäße. Diese Wirkung auf die Coronargefäße wird durch einen gleichzeitigen nitritähnlichen herzentlastenden Effekt verstärkt. Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. im Gehirn) manifestieren.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, die an der glatten des Magens, Darmtraktes, des Urogenitaltraktes und des Respirationssystems deutlich werden.

6. Die Verbindungen besitzen ebenfalls blutzuckersenkende Eigenschaften und zeigen Wirkung auf das zentrale Nervensystem.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide) synthetische Gesteinsmehle (z. B. hochdisperse Kieselsäure, Silikatel, Zucker (z. B. Roh-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer den obengenannten Hilfsstoffen mit verschiedenen Geschmacksaufbessern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht pro Tag zur Erzielung wirksamer Ergebnisse zu verabreichen, und bei oraler Applikation beträgt die Dosierung etwa 0,1 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Beispiel 1

6-Methyl-4-(2-nitrophenyl)-2-phenyl-1,4-dihydro-pyrimidin-5-carbonsäureethylester.

(Siehe Formel Seite 7 f.)

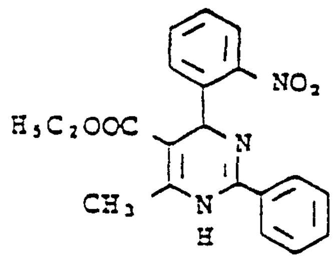

Verfahrensvariante A

15,8 g (60 mmol) 2-Nitrobenzylidenacetessigsäureethylester werden mit 11,5 g (66 mmol) Benzamidin-hydrochloridhydrat und 5,91 g (72 mmol) wasserfreiem Natriumacetat in 180 ml Ethanol 18 Stunden zum Rückfluß gekocht. Es wird abgekühlt und eingeengt. Der erhaltene Eindampfrückstand wird in einem Gemisch von Essigester, Wasser und 100 ml 1 N Salzsäure aufgenommen, gut durchgeschüttelt und getrennt. Die Essigesterphase wird noch einmal mit 100 ml 1 N Salzsäure extrahiert, die vereinten wäßrigen Phasen werden 1 x mit Ether ausgeschüttelt und anschließend mit konzentrierter Natronlauge alkalisch gestellt. Es wird 2 x mit Essigester extrahiert, die vereinten Essigesterphasen werden mit Wasser gewaschen, getrocknet und eingeengt. Das erhaltene Öl wird mit wenig kaltem Acetonitril verrührt ; die gebildeten Kristalle werden abgesaugt und mit kaltem Acetonitril gewaschen. Man erhält 12,2 g (55,71 % der Theorie) eines leicht gelb gefärbten Produktes vom Schmelzpunkt 102-105 °C.

## Beispiel 2

2,6-Dimethyl-4-(3-nitrophenyl)-1,4-dihydropyrimidin-5-carbonsäuremethylester-hydrochlorid

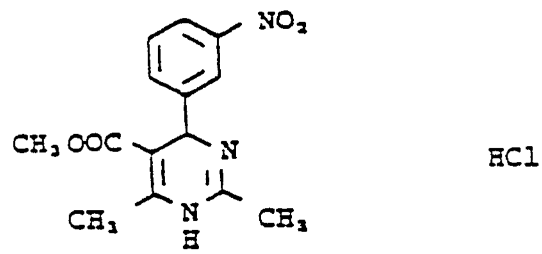

15 g (60 mmol) 3-Nitrobenzylidenacetessigsäuremethylester werden mit 6,3 g (66 mmol) Acetamidinhydrochlorid und 5,91 g (72 mmol) wasserfreiem Natriumacetat in 180 ml Ethanol 18 Stunden zum Rückfluß erhitzt. Es wird eingeengt. Der Eindampfrückstand wird mit Essigester versetzt und 2 x mit je 100 ml 1 N Salzsäure extrahiert. Die vereintein wäßrigen Phasen werden 1 x mit Ether ausgeschüttelt und danach mit konzentrierter Natronlauge alkalisch gestellt. Es wird 2 x mit Essigester extrahiert, die vereinten Essigesterphasen werden mit Wasser gewaschen, getrocknet und eingeengt. Der erhaltene ölige Eindampfrückstand (6,6 g) wird in Methanol gelöst und mit 23 ml 1 N Salzsäure versetzt. Es wird eingeengt, nochmals mit Ethanol eingeengt, die erhaltenen Kristalle werden mit Ethanol verrührt und abgesaugt. Man erhält 5,5 g farbloser Kristalle vom Schmelzpunkt 228 °C unter Zersetzung.

## Beispiel 3

6-Methyl-4-[2-(4-methylbenzyloxy)-phenyl]-2-phenyl-1,4-dihydro-pyrimidin-5-carbonsäureethylester-hydrochlorid

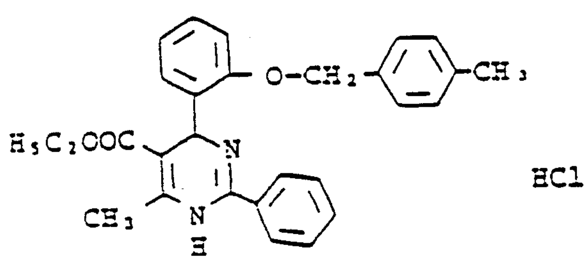

10,2 g (30 mmol) 2-(4-Methylbenzyloxy)-benzylidenacetessigsäureethylester werden mit 5,75 g (33 mmol) Benzamidin-hydrat-hydrochlorid und 2,95 g (36 mmol) wasserfreiem Natriumacetat in 90 ml Ethanol über Nacht zum Rückfluß gekocht. Es wird eingeengt ; der Eindampfrückstand wird mit 100 ml 1 N Salzsäure und 150 ml Ether versetzt und vier Stunden magnetisch gerührt, wobei das Hydrochlorid des Produktes ausfällt. Es wird abgesaugt, mit Wasser und Ether gewaschen und aus Acetonitril umkristallisiert. Man erhält 7,2 g (50,35 % der Theorie) eines nahezu farblosen Produktes vom Schmelzpunkt 223-26 °C unter Zersetzung.

Analog Verfahren A wurden hergestellt (siehe folgende Tabelle Seite 8 ff.).

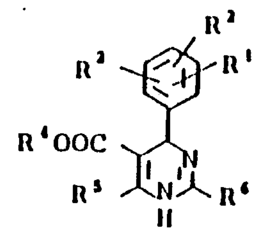

| Bsp. Nr. | $R^1$ | $R^2$ | $R^3$ | $R^4$ | $R^5$ | $R^6$ | Schmelzpunkt °C | Ausbeute % |
|---|---|---|---|---|---|---|---|---|
| 4 | 3-Cl | H | H | $CH_3$ | $CH_3$ | $CH_3$ | 211-13°C Z Hydrochlorid | 15.9 |
| 5 | 2-$NO_2$ | H | H | $CH_3$ | $CH_3$ | $CH_3$ | 164 | 19 |
| 6 | 3-Cl | H | H | $CH_3$ | $CH_3$ | H | 148-50 | 9.45 |
| 7 | 2-$NO_2$ | H | H | $CH_3$ | $CH_3$ | H | 237 | 19.4 |
| 8 | 2-$CH_3$-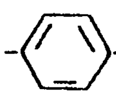-$CH_2$-O- | H | H | $CH_3$ | $CH_3$ | H | 130-132 | 4.76 |
| 9 | 2-$CH_3$-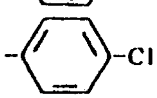-$CH_2$-O- | H | H | $C_2H_5$ | $CH_3$ | $CH_3$ | 164-65 | 11.46 |
| 10 | 2-$NO_2$ | H | H | $C_2H_5$ | $CH_3$ | $CH_3$ | 146 | 23.1 |
| 11 | 2-$NO_2$ | H | H | $C_2H_5$ | $CH_3$ | H | 162 | 21.9 |
| 12 | 3-Cl | H | H | $CH_3$ | $CH_3$ | $CH_3-CH_2-CH_2-$ | 131-32 | 4.85 |
| 13 | 3-$NO_2$ | H | H | $CH_3$ | $CH_3$ | $CH_3-CH_2-CH_2-$ | 149-51 | 23.66 |
| 14 | 2-$CF_3$ | H | H | $CH_3$ | $CH_3$ | $CH_3$ | 134-54 | 59.71 |

(Fortsetzung)

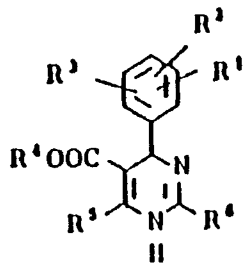

| Bsp. Nr. | $R^1$ | $R^2$ | $R^3$ | $R^4$ | $R^5$ | $R^6$ | Schmelzpunkt °C | Ausbeute % |
|---|---|---|---|---|---|---|---|---|
| 15 | 2-$CF_3$ | H | H | $CH_3$ | $CH_3$ | 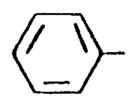 | 152-54 | 59.71 |
| 16 | 3-$NO_2$ | H | H | $CH_3$ | $CH_3$ | 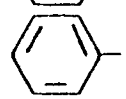 | 243-45 HCl-Salz | 77.85 |
| 17 | 3 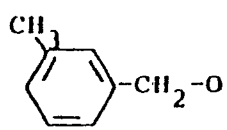 | 4-$CH_3$ | H | $CH_3$ | $CH_3$ | 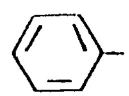 | 208-12 HCl-Salz | 22.31 |
| 18 | 2-$CF_3$ | H | H | $CH_3$ | $CH_3$ | H | 202-3 | 14.92 |

Beispiel 19

4-(3-Chlorphenyl)-6-methyl-2-phenyl-1,4-dihydro-pyrimidin-5-carbonsäuremethylester-hydrochlorid

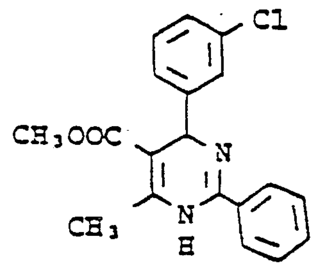

HCl

Verfahren B

4,21 g (30 mmol) 3-Chlorbenzaldehyd werden mit 3,45 g (30 mmol) β-Aminocrotonsäuremethylester, 5,22 g (30 mmol) Benzamidinhydrochlorid-hydrat und 2,95 g (36 mmol) wasserfreiem Natriumacetat in 90 ml Ethanol 16 Stunden gekocht. Es wird abgekühlt und eingeengt. Der Eindampfrückstand wird mit 75 ml Essigester und 50 ml 1 N Salzsäure versetzt und magnetisch gerührt. Das ausgefallene Salz wird abgesaugt, mit Essigester gewaschen und getrocknet. Man erhält 2,7 g leicht gelbgefärbte Kristalle vom Schmelzpunkt 237 °C unter Zersetzung.

Das Filtrat (Essigester und wäßrige Salzsäure) wird getrennt, die Essigesterphase einmal mit 50 ml 1 N Salzsäure ausgeschüttelt, die vereinten wäßrigen Phasen 1 mal mit Ether geschüttelt, getrennt und mit konzentrierter Natronlauge alkalisch gestellt. Es wird 2 x mit Essigester extrahiert, die vereinten Essigesterphasen werden mit Wasser gewaschen, getrocknet und eingeengt. Der Ölige Eindampfrückstand wird in Ethanol gelöst, mit 20 ml 1 N Salzsäure versetzt und eingeengt. Nach mehrmaligem Einengen mit Acetonitril werden die entstandenen Kristalle mit Acetonitril verrührt und abgesaugt. Man erhält 1,4 g leicht gelbgefärbter Kristalle vom Schmelzpunkt 237 °C unter Zersetzung, wodurch eine Gesamtausbeute von 4,1 g (36,25 % der Theorie) erhalten wird.

Verfahren C

4,21 g (30 mmol) 3-Chlorbenzaldehyd werden mit 3,24 ml (30 mmol) Acetessigsäuremethylester, 5,22 g (30 mmol) Benzamidin-hydrochlorid-hydrat und 2,95 ml (36 mmol) wasserfreiem Natriumacetat in 90 ml Ethanol 16 Stunden gekecht. Es wird abgekühlt und eingeengt. Der Eindampfrückstand wird mit 50 ml 1 N Salzsäure und 75 ml Essigester versetzt und magnetisch gerührt, wobei hellgelbe Kristalle ausfallen. Es wird abgesaugt und mit Essigester gewaschen. Man erhält 4,4 g leicht gelbgefärbter Kristalle vom Schmelzpunkt 236-38 °C unter Zersetzung. Aus dem Filtrat werden nach der bei Methode B beschriebenen Aufarbeitung noch 1,3 g der Verbindung erhalten, wodurch sich die Ausbeute auf 5,7 g (50,4 % der Theorie) erhöht.

Beispiel 20

4-(2-Chlorphenyl)-2,6-dimethyl-1,4-dihydro-pyrimidin-5-carbonsäuremethylester

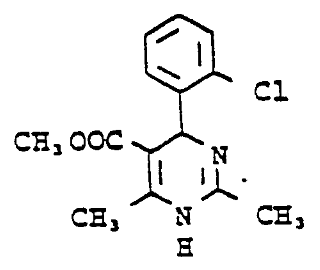

Verfahren C

8,42 g (60 mmol) 2-Chlorbenzaldehyd werden mit 6,48 ml (60 mmol) Acetessigsäuremethylester, 6,72 g (60 mmol) Acetamidin-HCl und 5,91 g (72 mmol) wasserfreiem Natriumacetat in 180 ml Ethanol 18 Stunden gekocht. Es wird abgekühlt und eingeengt. Der erhaltene Eindampfrückstand wird mit 100 ml 1 N Salzsäure und Essigester versetzt und gut durchgeschüttelt. Es wird getrennt, die Essigesterphase mit 100 ml 1 N Salzsäure ausgeschüttelt, die vereinten wäßrigen Phasen 1 x mit Ether ausgeschüttelt und getrennt. Die wäßrige Phase wird mit konzentrierter Natronlauge alkalisch gestellt und 2 x mit Essigester ausgeschüttelt. Die vereinten Essigesterphasen werden mit Wasser gewaschen, getrocknet und eingeengt.

Der kristalline Eindampfrückstand wird aus Acetonitril umkristallisiert. Man erhält 2,5 g einer leicht gelben Substanz vom Schmelzpunkt 225-27 °C.

Analog Verfahren C wurden hergestellt :

Beispiel 21

2-(4-Chlorphenyl)-4-(3-chlorphenyl)-6-methyl-1,4-dihydro-pyrimidin-5-carbonsäuremethylester-hydrochlorid vom Schmelzpunkt : 228 °C unter Zersetzung.

Beispiel 22

2-(3-Chlorphenyl)-4-(2-chlorphenyl)-6-methyl-1,4-dihydro-pyrimidin-5-carbonsäuremethylester-hydrochlorid vom Schmelzpunkt : 219-222 °C unter Zersetzung.

Beispiel 23

4-(2-Chlorphenyl)-6-methyl-2-(4-pyridyl)-1,4-dihydropyrimidin-5-carbonsäuremethylester vom Schmelzpunkt : 150 °C.

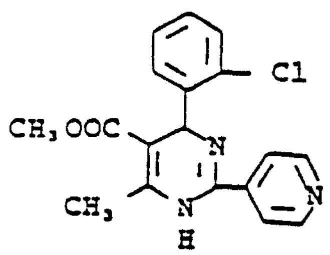

Beispiel 24

4-(2-Chlorphenyl)-6-methyl-2-(2-pyridyl)-1,4-dihydropyrimidin-5-carbonsäuremethylester vom Schmelzpunkt : 145 °C.

**Patentansprüche**

1. Dihydropyrimidine der allgemeinen Formel (I)

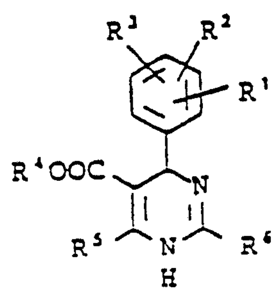

(I)

bzw. deren mesomeren Form der Formel (Ia)

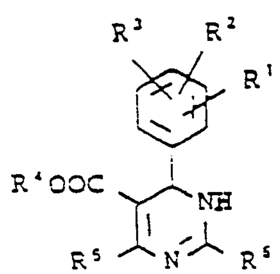

(Ia)

oder ein Gleichgewicht zwischen beiden Formen, in welcher $R^1$, $R^2$ und $R^3$ gleich oder verschieden sind und jeweils für Wasserstoff, Alkyl mit 1-4-Kohlenstoffatomen, Halogen, Nitro, Cyano, Hydroxy, Amino, Alkoxy mit 1-4 Kohlenstoffatomen ; Benzyl, Monoalkylamino oder Dialkylamino mit jeweils 1-4 Kohlenstoffatomen in den Alkylresten oder für Halogenalkyl mit 1-4 Kohlenstoffatomen, oder für den Rest

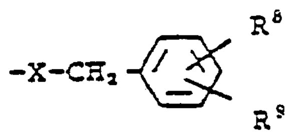

stehen, in welchem

X O, S oder $SO_2$ bedeutet und

$R^8$ und $R^9$ gleich oder verschieden sind und jeweils für Wasserstoff, Nitro, Trifluormethyl, Halogen, Alkyl oder Alkoxy mit jeweils 1-4 Kohlenstoffatomen stehen und

$R^4$ für Wasserstoff oder einem geradkettigen, verzweigten oder cyclischen Alkyl- oder Alkenylrest steht, der gegebenenfalls ein oder zwei gleiche oder verschiedene Heterokettenglieder aus der Gruppe O, CO, NH, N-Alkyl mit 1-4 Kohlenstoffatomen, S oder $SO_2$ enthält und der gegebenenfalls substituiert ist durch Halogen, Nitro, Cyano, Hydroxy, Phenyl, Benzyl, Pyridyl, Amino, Monoalkylamino oder Dialkylamino mit jeweils 1-4 Kohlenstoffatomen in den Alkylresten und

$R^5$ für einen geradkettigen, verzweigten oder cyclischen Alkyl- oder Alkenylrest mit bis zu 10 Kohlenstoffatomen steht, der gegebenenfalls substituiert ist durch Halogen, Hydroxy, Cyano, Amino, Alkoxy, Alkylthio, Alkoxycarbonyl, Monoalkylamino, Dialkylamino mit jeweils 1-4 Kohlenstoffatomen in den Alkyl- und Alkoxyresten, Phenyloxy, Benzylamino oder durch einen 5- bis 7-gliedrigen heterocyclischen Ring der ein oder zwei Heteroatome aus der Gruppe Sauerstoff oder Stickstoff als Ringglieder enthält, und

$R^6$ für Wasserstoff, einen geradkettigen, verzweigten oder cyclischen Alkyl- oder Alkenylrest mit bis zu 10 Kohlenstoffatomen steht der gegebenenfalls durch Halogen, Hydroxy, Cyano, Nitro, Amino, Alkoxy, Alkylthio, Alkoxycarbonyl, Monoalkylamino oder Dialkylamino mit jeweils 1-4 Kohlenstoffatomen in den Alkyl- und Alkoxyresten oder durch Phenoxy substituiert ist, oder für einen Phenyl-, Naphthyl-, Benzyl- oder Phenethylrest steht oder für einen stickstoffhaltigen Heteroarylrest steht, wobei die Arylreste gegebenenfalls 1- oder 2-fach substituiert sind durch Halogen, Hydroxy, Cyano, Amino, Nitro, Carboxy, Alkoxy, Alkylthio, Carbalkoxy, Acyloxy, Monoamino oder Dialkylamino mit jeweils bis zu 4 Kohlenstoffatomen in den Alkyl-, Alkoxy- und Acylresten, sowie ihre pharmakologisch unbedenklichen Additionssalze.

2. Dihydropyrimidine der allgemeinen Formel (I) gemäß Anspruch 1, in welcher $R^1$, $R^2$ und $R^3$ gleich oder verschieden sind und für Wasserstoff, Alkyl mit 1-4 Kohlenstoffatomen, Fluor, Chlor, Nitro, Trifluormethyl, oder für den Rest

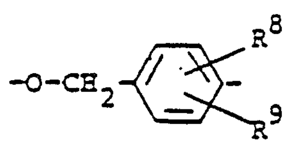

stehen, welchem

$R^8$ und $R^9$ gleich oder verschieden sind und für Wasserstoff, Alkyl mit 1-4 Kohlenstoffatomen, Fluor, Chlor, Nitro oder Trifluormethyl stehen,

$R^4$ und $R^5$ gleich oder verschieden sind und für Alkyl mit 1-6 Kohlenstoffatomen stehen, das gegebenenfalls in der Kette durch ein Sauerstoff unterbrochen ist und gegebenenfalls substituiert ist durch Fluor, Chlor, Cyano, Nitro und Phenyl, und

$R^6$ für Wasserstoff, Alkyl mit 1-4 Kohlenstoffatomen, Phenyl oder Pyridyl steht, sowie ihre pharmakologisch unbedenklichen Additionssalze.

3. Verfahren zur Herstellung von Dihydropyrimidinen der allgemeinen Formel (I)

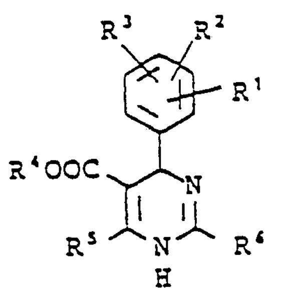

(I)

bzw. deren mesomeren Form der Formel (Ia)

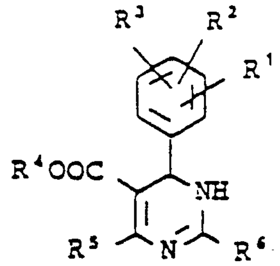

(Ia)

in welcher $R^1$, $R^2$ und $R^3$ gleich oder verschieden sind und jeweils für Wasserstoff, Alkyl mit 1-4 Kohlenstoffatomen, Halogen, Nitro, Cyano, Hydroxy, Amino, Alkoxy mit 1-4 Kohlenstoffatomen ; Benzyl, Monoalkylamino oder Dialkylamino mit jeweils 1-4 Kohlenstoffatomen in den Alkylresten oder für Halogenalkyl mit 1-4 Kohlenstoffatomen, oder für den Rest

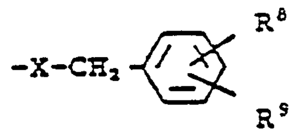

stehen, in welchem

X O, S oder $SO_2$ bedeutet und

$R^8$ und $R^9$ gleich oder verschieden sind und jeweils für Wasserstoff, Nitro, Trifluormethyl, Halogen, Alkyl oder Alkoxy mit jeweils 1-4 Kohlenstoffatomen stehen und

$R^4$ für Wasserstoff oder einen geradkettigen, verzweigten oder cyclischen Alkyl- oder Alkenylrest steht, der gegebenenfalls ein oder zwei gleiche oder verschiedene Heterokettenglieder aus der Gruppe O, CO, NH, N-Alkyl mit 1-4 Kohlenstoffatomen, S oder $SO_2$ enthält und der gegebenenfalls substituiert ist durch Halogen, Nitro, Cyano, Hydroxy, Phenyl, Benzyl, Pyridyl, Amino, Monoalkylamino oder Dialkylamino mit jeweils 1-4 Kohlenstoffatomen in den Alkylresten und

$R^5$ für einen geradkettigen, verzweigten oder cyclischen Alkyl- oder Alkenylrest mit bis zu 10 Kohlenstoffatomen steht, der gegebenenfalls substituiert ist durch Halogen, Hydroxy, Cyano, Amino, Alkoxy, Alkylthio, Alkoxycarbonyl, Monoalkylamino, Dialkylamino mit jeweils 1-4 Kohlenstoffatomen in den Alkyl- und Alkoxyresten, Phenyloxy, Benzylamino oder durch einen 5- bis 7-gliedrigen heterocyclischen Ring der ein oder zwei Heteroatome aus der Gruppe Sauerstoff oder Stickstoff als Ringglieder enthält, und

$R^6$ für Wasserstoff, einen geradkettigen, verzweigten oder cyclischen Alkyl- oder Alkenylrest mit bis zu 10 Kohlenstoffatomen steht der gegebenenfalls durch Halogen, Hydroxy, Cyano, Nitro, Amino, Alkoxy, Alkylthio, Alkoxycarbonyl, Monoalkylamino oder Dialkylamino mit jeweils 1-4 Kohlenstoffatomen in den Alkyl- und Alkoxyresten oder durch Phenoxy substituiert ist, oder für einen Phenyl-, Naphthyl-, Benzyl- oder Phenethylrest steht oder für einen stickstoffhaltigen Heteroarylrest steht, wobei die Arylreste gegebenenfalls 1- oder 2-fach substituiert sind durch Halogen, Hydroxy, Cyano, Amino, Nitro, Carboxy, Alkoxy, Alkylthio, Carbalkoxy, Acyloxy, Monoamino oder Dialkylamino mit jeweils bis zu 4 Kohlenstoffatomen in den Alkyl-, Alkoxy- und Acylresten, sowie ihrer pharmakologisch unbedenklichen Additionssalze, dadurch gekennzeichnet, daß man .

A) Yliden-β-ketoester der allgemeinen Formel (II)

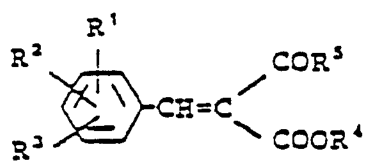

(II)

in welcher $R^1$, $R^2$, $R^3$, $R^4$ und $R^5$ die oben angegebene Bedeutung haben, mit Amidinen der allgemeinen Formel (III)

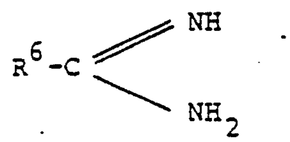

(III)

in welcher $R^6$ die oben angegebene Bedeutung hat, gegebenenfalls in Gegenwart inerter organischer Lösungsmittel bei Temperaturen zwischen 20° und 150 °C mit oder ohne Basen- oder Säurezusatz umsetzt, oder

B) Aldehyde der allgemeinen Formel (IV)

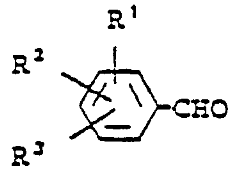

(IV)

in welcher $R^1$, $R^2$ und $R^3$ die oben angegebene Bedeutung haben, mit Enaminocarbonsäureestern der allgemeinen Formel (V)

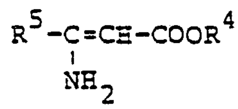

(V)

in welcher $R^4$ und $R^5$ die oben angegebene Bedeutung haben, mit Amidinen der allgemeinen Formel (III) wie oben beschrieben umsetzt, oder

C) Aldehyde der allgemeinen Formel (IV)

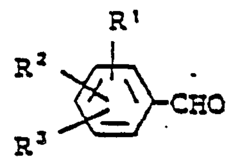

(IV)

in welcher $R^1$, $R^2$ und $R^3$ die oben angegebene Bedeutung haben, mit Amidinen der Formel (III)

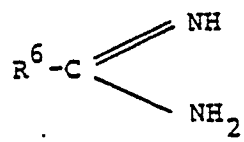

(III)

in welcher $R^6$ die oben angegebene Bedeutung hat und β-Ketocarbonsäureestern der allgemeinen Formel (VI)

$$R^5—CO—CH_2—COOR^4 \quad (VI)$$

in welcher $R^4$ und $R^5$ die oben angegebene Bedeutung haben, gegebenenfalls in Gegenwart inerter organischer Lösungsmittel mit oder ohne Basen- bzw. Säurezusatz umsetzt.

4. Dihydropyrimidine der allgemeinen Formel (I) gemäß Anspruch 1 zur Verwendung bei der Bekämpfung von Erkrankungen.

5. Dihydropyrimidine der allgemeinen Formel (I) gemäß Anspruch 1 zur Verwendung bei der Bekämpfung von Kreislauferkrankungen.

6. Arzneimittel enthaltend mindestens eine Verbindung der allgemeinen Formel (I) gemäß Anspruch 1.

7. Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

8. Verwendung von Verbindungen der allgemeinen Formel (I) gemäß Anspruch 1 bei der Herstellung von Kreislaufmitteln.

**Claims**

1. Dihydropyrimidines of the general formula (I)

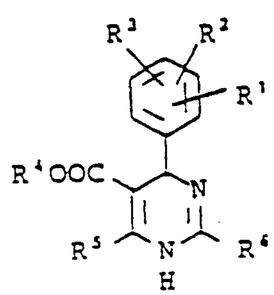

(I)

and the mesomeric form thereof of the formula (Ia)

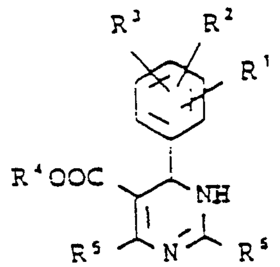

(Ia)

or an equilibrium between the two forms, in which $R^1$, $R^2$ and $R^3$ are identical or different and in each case represent hydrogen, alkyl with 1-4 carbon atoms, halogen, nitro, cyano, hydroxyl, amino, alkoxy with 1-4 carbon atoms, benzyl, monoalkylamino or dialkylamino with in each case 1-4 carbon atoms in the alkyl radicals or halogenoalkyl with 1-4 carbon atoms, or the radical

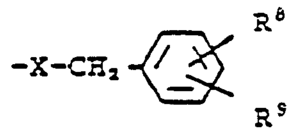

in which

X denotes O, S or $SO_2$ and

$R^8$ and $R^9$ are identical or different and each represent hydrogen, nitro, trifluoromethyl, halogen, or alkyl or alkoxy with in each case 1-4 carbon atoms and

$R^4$ represents hydrogen or a straight-chain, branched or cyclic alkyl or alkenyl radical which optionally contains one or two identical or different hetero chain members from the group comprising O, CO, NH, N-alkyl with 1-4 carbon atoms, S or $SO_2$ and which is optionally substituted by halogen, nitro, cyano, hydroxyl, phenyl, benzyl, pyridyl, amino, or monoalkylamino or dialkylamino with in each case 1-4 carbon atoms in the alkyl radicals and

$R^5$ represents a straight-chain, branched or cyclic alkyl or alkenyl radical which has up to 10 carbon atoms and is optionally substituted by halogen, hydroxyl, cyano, amino, alkoxy, alkylthio, alkoxycarbonyl, monoalkylamino or dialkylamino with in each case 1-4 carbon atoms in the alkyl and alkoxy radicals, phenyloxy, benzylamino or by a 5- to 7-membered heterocyclic ring which contains one or two hetero atoms from the group comprising oxygen and nitrogen as ring members, and

$R^6$ represents hydrogen, a straight-chain, branched or cyclic alkyl or alkenyl radical which has up to 10 carbon atoms and is optionally substituted by halogen, hydroxyl, cyano, nitro, amino, alkoxy, alkylthio, alkoxycarbonyl, monoalkylamino or dialkylamino with in each case 1-4 carbon atoms in the alkyl and alkoxy radicals or by phenoxy, or represents a phenyl, naphthyl, benzyl or phenethyl radical or represents a nitrogen-containing heteroaryl radical, the aryl radicals optionally being mono- or disubstituted by halogen, hydroxyl, cyano, amino, nitro, carboxyl, alkoxy, alkylthio, carbalkoxy, acyloxy, monoamino or dialkylamino with in each case up to 4 carbon atoms in the alkyl, alkoxy and acyl radicals, and their pharmacologically acceptable addition salts.

2. Dihydropyrimidines of the general formula (I) according to Claim 1, in which $R^1$, $R^2$ and $R^3$ are identical or different and represent hydrogen, alkyl with 1-4 carbon atoms, fluorine, chlorine, nitro, trifluoromethyl, or the radical

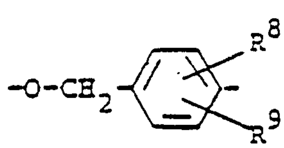

in which

$R^8$ and $R^9$ are identical or different and represent hydrogen, alkyl with 1-4 carbon atoms, fluorine, chlorine, nitro or trifluoromethyl,

$R^4$ and $R^5$ are identical or different and represent alkyl with 1-6 carbon atoms which is optionally interrupted in the chain by one oxygen and is optionally substituted by fluorine, chlorine, cyano, nitro and phenyl and

$R^6$ represents hydrogen, alkyl with 1-4 carbon atoms, phenyl or pyridyl, and their pharmacologically acceptable addition salts.

3. Process for the preparation of dihydropyrimidines of the general formula (I)

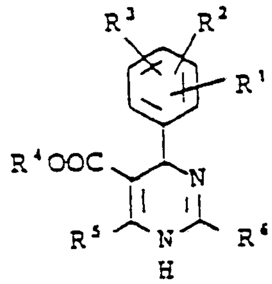

(I)

and the mesomeric form thereof of the formula (Ia)

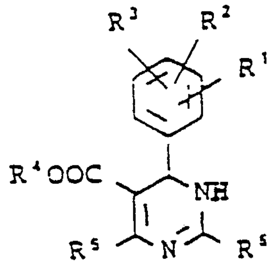

(Ia)

in which $R^1$, $R^2$ and $R^3$ are identical or different and in each case represent hydrogen, alkyl with 1-4 carbon atoms, halogen, nitro, cyano, hydroxyl, amino, alkoxy with 1-4 carbon atoms, benzyl, monoalkylamino or dialkylamino with in each case 1-4 carbon atoms in the alkyl radicals, or halogenoalkyl with 1-4 carbon atoms, or the radical

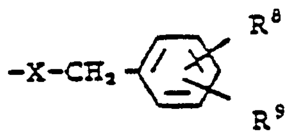

in which

X denotes O, S or $SO_2$ and

$R^8$ and $R^9$ are identical or different and each represent hydrogen, nitro, trifluoromethyl, halogen, or alkyl or alkoxy with in each case 1-4 carbon atoms and

$R^4$ represents hydrogen or a straight-chain, branched or cyclic alkyl or alkenyl radical which optionally contains one or two identical or different hetero chain members from the group comprising O, CO, NH, N-alkyl with 1-4 carbon atoms, S or $SO_2$ and which is optionally substituted by halogen, nitro, cyano, hydroxyl, phenyl, benzyl, pyridyl, amino, or monoalkylamino or dialkylamino with in each case 1-4 carbon atoms in the alkyl radicals and

$R^5$ represents a straight-chain, branched or cyclic alkyl or alkenyl radical which has up to 10 carbon atoms and is optionally substituted by halogen, hydroxyl, cyano, amino, alkoxy, alkylthio, alkoxycarbonyl, monoalkylamino or dialkylamino with in each case 1-4 carbon atoms in the alkyl and alkoxy radicals, phenyloxy, benzylamino or by a 5- to 7-membered heterocyclic ring which contains one or two hetero atoms from the group comprising oxygen and nitrogen as ring members, and

$R^6$ represents hydrogen, a straight-chain, branched or cyclic alkyl or alkenyl radical which has up to 10 carbon atoms and is optionally substituted by halogen, hydroxyl, cyano, nitro, amino, alkoxy, alkylthio, alkoxycarbonyl, monoalkylamino or dialkylamino with each case 1-4 carbon atoms in the alkyl and alkoxy radicals or by phenoxy, or represents a phenyl, naphthyl, benzyl or phenethyl radical or represents a nitrogen-containing heteroaryl radical, the aryl radicals optionally being mono- or disubstituted by halogen, hydroxyl, cyano, amino, nitro, carboxyl, alkoxy, alkylthio, carbalkoxy, acyloxy, monoamino or dialkylamino with in each case up to 4 carbon atoms in the alkyl, alkoxy and acyl radicals, and their pharmacologically acceptable addition salts, characterised in that

A) ylidene-β-keto esters of the general formula (II)

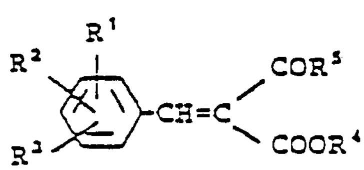

(II)

in which $R^1$, $R^2$, $R^3$, $R^4$ and $R^5$ have the abovementioned meaning, are reacted with amidines of the general formula (III)

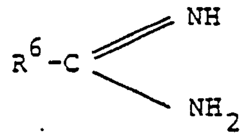

(III)

in which $R^6$ has the abovementioned meaning, if appropriate in the presence of inert organic solvents, at temperatures between 20° and 150 °C, with or without the addition of a base or acid, or

B) aldehydes of the general formula (IV)

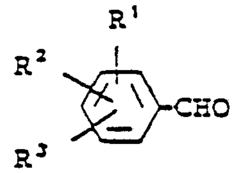

(IV)

in which $R^1$, $R^2$ and $R^3$ have the abovementioned meaning, are reacted with enaminocarboxylic acid esters of the general formula (V)

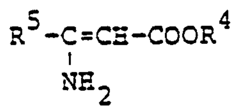

(V)

in which $R^4$ and $R^5$ have the abovementioned meaning, with amidines of the general formula (III), as described above, or

C) aldehydes of the general formula (IV)

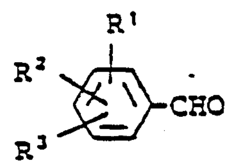

(IV)

in which $R^1$, $R^2$ and $R^3$ have the abovementioned meaning, are reacted with amidines of the formula (III)

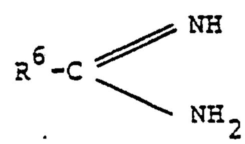

(III)

in which $R^6$ has the abovementioned meaning and β-ketocarboxylic acid esters of the general formula (VI)

$$R^5—CO—CH_2—COOR^4 \qquad (VI)$$

in which $R^4$ and $R^5$ have the abovementioned meaning, if appropriate in the presence of inert organic solvents, with or without the addition of a base or acid.

4. Dihydropyrimidines of the general formula (I) according to Claim 1 for use in combating diseases.

5. Dihydropyrimidines of the general formula (I) according to Claim 1 for use in combating circulatory diseases.

6. Medicaments containing at least one compound of the general formula (I) according to Claim 1.

7. Process for the preparation of medicaments, characterised in that compounds of the general formula (I) according to Claim 1 are converted into a suitable administration form, if appropriate using customary auxiliaries and excipients.

8. Use of compounds of the general formula (I) according to Claim 1 in the preparation of circulatory agents.

**Revendications**

1. Dihydropyrimidines de formule générale (I)

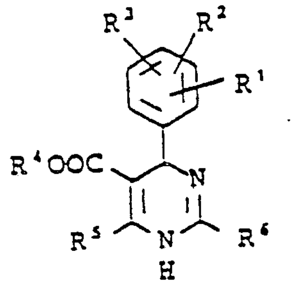

(I)

ou leur forme mésomérique de formule (Ia)

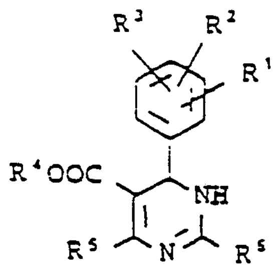

(Ia)

ou un équilibre entre les deux formes, formules dans lesquelles $R^1$, $R^2$ et $R^3$ sont identiques ou différentes et représentent chacun l'hydrogène, un groupe alkyle ayant 1 à 4 atomes de carbone, un halogène, un groupe nitro, cyano, hydroxy, amino, alkoxy ayant 1 à 4 atomes de carbone, benzyle, monoalkylamino ou dialkylamino ayant chacun 1 à 4 atomes de carbone dans les restes alkyle ou un groupe halogénalkyle ayant 1 à 4 atomes de carbone, ou le reste

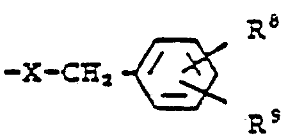

dans lequel

X représente O, S ou $SO_2$ et

$R^8$ et $R^9$ sont identiques ou différents et représentent chacun l'hydrogène ou un groupe nitro, trifluorométhyle, un halogène, un groupe alkyle ou un groupe alkoxy ayant chacun 1 à 4 atomes de carbone et

$R^4$ représente l'hydrogène ou un reste alkyle ou alcényle à chaîne droite, ramifié ou cyclique, qui comprend éventuellement un ou deux maillons hétéro-atomiques identiques ou différents du groupe O, CO, NH, N-alkyle avec 1 à 4 atomes de carbone, S ou $SO_2$ et qui est substitué le cas échéant par un halogène, un groupe nitro, cyano, hydroxy, phényle, benzyle, pyridyle, amino, monoalkylamino ou dialkylamino ayant dans chaque cas 1 à 4 atomes de carbone dans les restes alkyle et

$R^5$ représente un reste alkyle ou alcényle à chaîne droite, ramifié ou cyclique ayant jusqu'à 10 atomes de carbone, qui est substitué le cas échéant par un halogène ou un groupe hydroxy, cyano, amino, alkoxy, alkylthio, alkoxycarbonyle, monoalkylamino, dialkylamino avec dans chaque cas 1 à 4 atomes de carbone dans les restes alkyle et alkoxy, un groupe phényloxy, benzylamino ou par un noyau hétérocyclique pentagonal à heptagonal qui comporte comme chaînons un ou deux hétéro-atomes du groupe oxygène ou azote, et

$R^6$ représente l'hydrogène, un reste alkyle ou alcényle à chaîne droite, ramifié ou cyclique ayant jusqu'à 10 atomes de carbone, qui est substitué le cas échéant par un halogène, un groupe hydroxy, cyano, nitro, amino, alkoxy, alkylthio, alkoxycarbonyle, monoalkylamino ou dialkylamino ayant dans chaque cas 1 à 4 atomes de carbone dans les restes alkyle et alkoxy ou par un groupe phénoxy, ou représente un reste phényle, naphtyle, benzyle ou phénéthyle ou un reste hétéro-aryle azoté, les restes aryle étant substitués le cas échéant une ou deux fois par un substituant halogéno, hydroxy, cyano, amino, nitro, carboxy, alkoxy, alkylthio, carbalkoxy, acyloxy, monoalkylamino ou dialkylamino ayant dans chaque cas jusqu'à 4 atomes de carbone dans les restes alkyle, alkoxy et acyle, ainsi que leurs sels d'addition pharmacologiquement acceptables.

2. Dihydropyrimidines de formule générale (I) suivant la revendication 1, dans laquelle $R^1$, $R^2$ et $R^3$

sont identiques ou différents et représentent l'hydrogène, un groupe alkyle ayant 1 à 4 atomes de carbone, le fluor, le chlore, un groupe nitro, trifluorométhyle ou le reste de formule

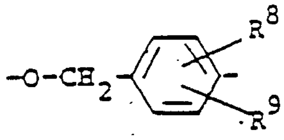

dans laquelle

$R^8$ et $R^9$ sont identiques ou différents et représentent l'hydrogène, un groupe alkyle ayant 1 à 4 atomes de carbone, le fluor, le chlore, un groupe nitro ou trifluorométhyle,

$R^4$ et $R^5$ sont identiques ou différents et représentent un groupe alkyle ayant 1 à 6 atomes de carbone, dont la chaîne est interrompue le cas échéant par un atome d'oxygène et qui est éventuellement substitué par du fluor, du chlore, des groupes cyano, nitro et phényle, et

$R^6$ représente l'hydrogène, un groupe alkyle ayant 1 à 4 atomes de carbone, phényle ou pyridyle, ainsi que leurs sels d'addition pharmacologiquement acceptables.

3. Procédé de production de dihydropyrimidines de formule générale (I)

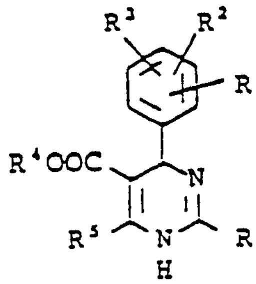

(I)

et de leur forme mésomérique de formule (Ia)

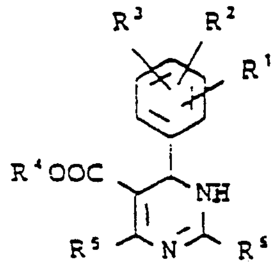

(Ia)

formules dans lesquelles $R^1$, $R^2$ et $R^3$ sont identiques ou différents et représentent chacun l'hydrogène, un groupe alkyle ayant 1 à 4 atomes de carbone, un halogène, un groupe nitro, cyano, hydroxy, amino, alkoxy ayant 1 à 4 atomes de carbone, benzyle, monoalkylamino ou dialkylamino ayant chacun 1 à 4 atomes de carbone dans les restes alkyle ou un groupe halogénalkyle ayant 1 à 4 atomes de carbone, ou le reste

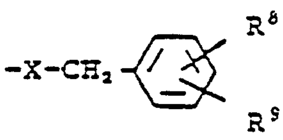

dans lequel

X représente O, S ou $SO_2$ et

$R^8$ et $R^9$ sont identiques ou différents et représentent chacun l'hydrogène ou un groupe nitro, trifluorométhyle, un halogène, un groupe alkyle ou un groupe alkoxy ayant chacun 1 à 4 atomes de carbone et

$R^4$ représente l'hydrogène ou un reste alkyle ou alcényle à chaîne droite, ramifié ou cyclique, qui comprend éventuellement un ou deux maillons hétéro-atomiques identiques ou différents du groupe O, CO, NH, N-alkyle avec 1 à 4 atomes de carbone, S ou $SO_2$ et qui est substitué le cas échéant par un halogène, un groupe nitro, cyano, hydroxy, phényle, benzyle, pyridyle, amino, monoalkylamino ou dialkylamino ayant dans chaque cas 1 à 4 atomes de carbone dans les restes alkyle et

$R^5$ représente un reste alkyle ou alcényle à chaîne droite, ramifié ou cyclique ayant jusqu'à 10 atomes de carbone, qui est substitué le cas échéant par un halogène ou un groupe hydroxy, cyano, amino,

alkoxy, alkylthio, alkoxycarbonyle, monoalkylamino, dialkylamino avec dans chaque cas 1 à 4 atomes de carbone dans les restes alkyle et alkoxy, un groupe phényloxy, benzylamino ou par un noyau hétérocyclique pentagonal à heptagonal qui comporte comme chaînons un ou deux hétéro-atomes du groupe oxygène ou azote, et

$R^6$ représente l'hydrogène, un reste alkyle ou alcényle à chaîne droite, ramifié ou cyclique ayant jusqu'à 10 atomes de carbone, qui est substitué le cas échéant par un halogène, un groupe hydroxy, cyano, nitro, amino, alkoxy, alkylthio, alkoxycarbonyle, monoalkylamino ou dialkylamino ayant dans chaque cas 1 à 4 atomes de carbone dans les restes alkyle et alkoxy ou par un groupe phénoxy, ou représente un reste phényle, naphtyle, benzyle ou phénéthyle ou un reste hétéro-aryle azoté, les restes aryle étant substitués le cas échéant une ou deux fois par un substituant halogéno, hydroxy, cyano, amino, nitro, carboxy, alkoxy, alkylthio, carbalkoxy, acyloxy, monoalkylamino ou dialkylamino ayant dans chaque cas jusqu'à 4 atomes de carbone dans les restes alkyle, alkoxy et acyle, ainsi que de leurs sels d'addition pharmacologiquement acceptables, caractérisé en ce que :

A) on fait réagir des yliden-β-cétoesters de formule générale (II)

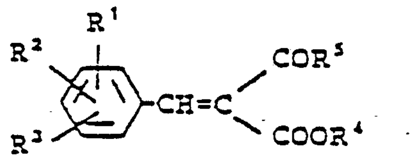

(II)

dans laquelle $R^1$, $R^2$, $R^3$, $R^4$ et $R^5$ ont la définition indiquée ci-dessus, avec des amidines de formule générale (III)

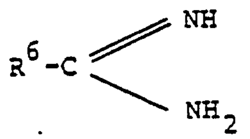

(III)

dans laquelle $R^6$ a la définition indiquée ci-dessus, éventuellement en présence de solvants organiques inertes à des températures comprises entre 20 et 150 °C avec ou sans addition de bases ou d'acides, ou bien

B) on fait réagir des aldéhydes de formule générale (IV)

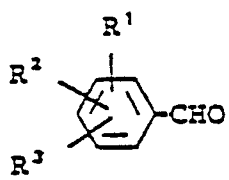

(IV)

dans laquelle $R^1$, $R^2$ et $R^3$ ont la définition indiquée ci-dessus, avec des esters d'acides énaminocarboxyliques de formule générale (V)

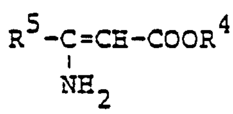

(V)

dans laquelle $R^4$ et $R^5$ ont la définition indiquée ci-dessus, avec des amidines de la formule générale (III), de la manière décrite ci-dessus, ou bien

C) on fait réagir des aldéhydes de formule générale (IV)

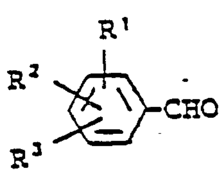

(IV)

dans laquelle $R^1$, $R^2$ et $R^3$ ont la définition indiquée ci-dessus, avec des amidines de formule (III)

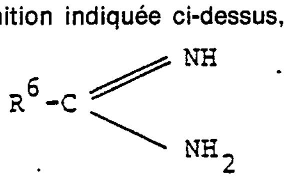

(III)

dans laquelle $R^6$ a la définition indiquée ci-dessus, et des esters d'acides β-cétocarboxyliques de formule générale (VI)

$$R^5—CO—CH_2—COOR^4 \qquad (VI)$$

dans laquelle $R^4$ et $R^5$ ont la définition indiquée ci-dessus, le cas échéant en présence de solvants organiques inertes, avec ou sans addition de bases ou d'acides.

4. Dihydropyrimidines de formule générale (I) suivant la revendication 1, destinées à être utilisées pour combattre des maladies.

5. Dihydropyrimidines de formule générale (I) suivant la revendication 1, destinées à être utilisées pour combattre des maladies de la circulation.

6. Médicaments contenant au moins un composé de formule générale (I) suivant la revendication 1.

7. Procédé de préparation de médicaments, caractérisé en ce qu'on transforme des composés de formule générale (I) suivant la revendication 1, éventuellement en utilisant des adjuvants et des supports classiques, en une forme propre à l'administration.

8. Utilisation de composés de formule générale (I) suivant la revendication 1 dans la préparation d'agents influençant la circulation.